Europäisches Patentamt

European Patent Office

Office européen des brevets

(11) Veröffentlichungsnummer: **0 360 078**
**A2**

(12) ## EUROPÄISCHE PATENTANMELDUNG

(21) Anmeldenummer: 89116499.8

(22) Anmeldetag: 07.09.89

(51) Int. Cl.⁵: **A61B 10/00**

(30) Priorität: 21.09.88 DE 3831967

(43) Veröffentlichungstag der Anmeldung:
28.03.90 Patentblatt 90/13

(84) Benannte Vertragsstaaten:
AT BE CH DE ES FR GB GR IT LI LU NL SE

(71) Anmelder: Menton, Michael, Dr.
Ursrainer Ring 103
D-7400 Tübingen(DE)

(72) Erfinder: Menton, Michael, Dr.
Ursrainer Ring 103
D-7400 Tübingen(DE)

(74) Vertreter: Reimold, Otto, Dr. Dipl.-Phys. et al
Patentanwälte Dipl.-Ing. R. Magenbauer
Dipl.-Phys. Dr. O. Reimold Dipl.-Phys.Dr. H.
Vetter Hölderlinweg 58
D-7300 Esslingen(DE)

(54) **Vorrichtung zum operativen Entfernen eines Gewebeteils aus einem menschlichen oder tierischen Körper.**

(57) Bei einer Vorrichtung (10) zum operativen Entfernen eines kegelähnliche Gestalt aufweisenden und eine Öffnung (6) enthaltenden Gewebeteils (9) aus einem menschlichen oder tierischen Körper durch Ausschneiden mittels eines Laserstrahls ist ein Laserstrahl-Führungsgehäuse (11) vorhanden. Dieses Führungsgehäuse (11) weist ein Rohr (20), durch das der eintretende Laserstrahl verläuft, sowie ein den Laserstrahl aus seiner Eintrittsrichtung (15) weg lenkendes eintrittsseitiges Umlenkteil (17) und ein mit Seitenabstand zu diesem angeordnetes, den Laserstrahl seine Austrittsrichtung (16) lenkendes austrittsseitiges Umlenkteil (18) auf, so daß beim Verdrehen des Führungsgehäuses (11) um die Eintrittsrichtung (15) der austretende Laserstrahl (16) eine Kegelmantelfläche überstreicht. Das austrittsseitige Umlenkteil (18) ist an einem seitlich vom Rohr (20) abstehenden Auslegeteil (21) des Führungsgehäuses (11) angeordnet. Die Vorrichtung (10) weist ferner einen in die Öffnung (6) steckbaren und das Führungsgehäuse (11) beim Verdrehen zentrierenden Führungsstift (28) auf, wobei der Führungsstift (28) und das Führungsgehäuse (11) gegeneinander verdrehbar sind.

FIG. 1

# Vorrichtung zum operativen Entfernen eines Gewebeteils aus einem menschlichen oder tierischen Körper

Die Erfindung betrifft eine Vorrichtung zum operativen Entfernen eines kegelähnliche Gestalt aufweisenden und eine Öffnung enthaltenden Gewebeteils aus einem menschlichen oder tierischen Körper durch Ausschneiden mittels eines Laserstrahls, mit einem Laserstrahl-Führungsgehäuse, das ein Rohr, durch das der eintretende Laserstrahl verläuft, sowie ein den Laserstrahl aus seiner Eintrittsrichtung weglenkendes eintrittseitiges Umlenkteil und ein mit Seitenabstand zu diesem angeordnetes, den Laserstrahl in seine Austrittsrichtung lenkendes austrittseitiges Umlenkteil aufweist, so daß beim Verdrehen des Führungsgehäuses um die Eintrittsrichtung der austretende Laserstrahl eine Kegelmantelfläche überstreicht, und mit einem in die Öffnung steckbaren und das Führungsgehäuse beim Verdrehen zentrierenden Führungsstift.

Eine Gebärmutter besteht in ihren Hauptbestandteilen aus dem Gebärmutterkörper (Corpus uteri) und dem Gebärmutterhals (Cervix uteri). Dabei ragt die Gebärmutter mit dem Gebärmutterhals wenige Zentimeter in die Scheide. Dieser unterste Abschnitt des Gebärmutterhalses wird als Gebärmuttermund (Portio) bezeichnet. Im Bereich des Gebärmuttermundes befindet sich histologisch der Übergang zwischen der Gebärmutterschleimhaut, die die gesamte Gebärmutterhöhle auskleidet ,und einem Deckgewebe (Plattenepithel), das den in die Scheide hineinragenden Teil der Gebärmutter bedeckt. Diese Übergangszone bezeichnet man als Transformationszone, die ein Regenerationsbereich ist, in dem die Zelldifferenzierung stattfindet. Auch atypische Zellen gehen von diesem Regenerationsbereich aus und können nach einer gewissen Latenzzeit in bösartiges Wachstum übergehen. Die Areale, von denen solche Zellveränderungen ausgehen, sind meist durch Spezialuntersuchungen (Kolposkopie) am Gebärmutterhals zu lokalisieren. Bei rechtzeitiger Diagnosestellung können diese präinvasiven Stadien des Gebärmutterhalskarzinoms durch das Ausschneiden eines je nach Befund kleineren oder größeren Kegels oder Konus entfernt werden. Ein solcher Eingriff ermöglicht somit die Erhaltung der Gebärmutter, was insbesondere bei jungen Patientinnen zur Erhaltung der Fruchtbarkeit (Fertilität) von herausragender Bedeutung ist.

Zur Durchführung dieses Eingriffs ist in der DE-OS 23 09 205 bereits eine Vorrichtung der eingangs genannten Art vorgeschlagen worden. Bei ihrer Anwendung wird sie in die Scheide eingeführt und mit dem Führungsstift in die Gebärmutterhalsöffnung gesteckt. Nach dem Einschalten der Laser-Quelle wird das Rohr und mit diesem das eintrittseitige sowie das austrittseitige Umlenkteil um die Rohrachse gedreht, so daß der gewünschte Gewebekegel ausgeschnitten wird. Gegenüber dem üblichen Ausschneiden mit Hilfe eines Messers ergibt sich hierdurch eine wesentliche Verkürzung der Operationszeit auf wenige Minuten. Ferner ist der Blutverlust sehr gering und Nachblutungen treten praktisch keine auf. Desweiteren sind keine Folgeerscheinungen, wie verringerte Fruchtbarkeit zu erwarten. Außerdem kann die Operation aufgrund der gegenüber dem chirurgischen Verfahren, bei dem ein stationärer Krankenhausaufenthalt von etwa 8 Tagen erforderlich ist, vernachlässigbaren Komplikationsrate auch ambulant durchgeführt werden. Ein weiterer Vorteil einer solchen Laser-Konisation liegt darin, daß sich der ausgeschnittene Kegel postoperativ gut pathologisch untersuchen läßt.

Neben diesen Vorzügen dieser Laser-Konisation weist die bekannte Vorrichtung jedoch auch Nachteile auf:

Das die als Spiegel ausgebildeten Umlenkteile enthaltende Rohr ist seinerseits in einem feststehenden Außenrohr geführt. Diese Bauform ist deshalb zwangsläufig rotationssymmetrisch und nimmt an der Operationsstelle rundum viel Platz in Anspruch, so daß diese nur mittels eines Mikroskopes durch das Rohr hindurch betrachtet werden kann. Dabei ist nur die momentane Laserschnittstelle beobachtbar, da der Mikroskop-Lichtstrahl über die gleichen Spiegel zur Operationsstelle gelenkt wird. Außerdem wird wegen der beengten Platzverhältnisse der instrumentelle Zugang zur Operationsstelle behindert.

Ein weiterer Nachteil besteht in der festen Anordnung des Führungsstiftes am Führungsgehäuse. Daher tritt zwischen dem sich beim Herausschneiden des Gewebekegels mitdrehenden Führugnsstift und dem ihn umgebenden Gewebe eine Reibungskraft auf, so daß der herauszuschneidende Gewebekegel auf Torsion beansprucht wird. Dies kann eine ungenaue Kegelform ergeben und zu einem Abreißen des Gewebekegels kurz vor Beendigung des Ausschneidvorgangs führen. Ferner leidet unter den auftretenden Kräften die Zentrierung des Führungsstiftes, was sich ebenfalls ungünstig auf die erhaltene Kegelform auswirkt.

Dieser Nachteil tritt vor allem dann auf, wenn das Gerät nur mit der Hand gehalten und nicht mit ortsfest angebrachtem Außenrohr - in diesem Falle übernimmt das Außenrohr die Zentrierfunktion - betrieben wird.

Der vorliegenden Erfindung liegt deshalb die Aufgabe zugrunde, eine Vorrichtung der eingangs genannten Art zu schaffen, die unter Beibehaltung der geschilderten Vorteile bei ihrer Anwendung eine bessere Betrachtung der Operationsstelle zuläßt und das Zuführen anderer Instrumente weniger behindert, wobei außerdem die mit dem Führungsstift erreichte Zentrierung verbessert und das Auftreten von Reibungskräften am herauszuschneidenden Kegel vermieden werden soll.

Diese Aufgabe wird erfindungsgemäß dadurch gelöst, daß das Führungsgehäuse ein seitlich vom Rohr abstehendes Auslegerteil aufweist, an dem das austrittseitige Umlenkteil angeordnet ist und daß der Führungsstift und das Führungsgehäuse gegeneinander verdrehbar sind.

Aufgrund des Auslegerteils ergibt sich eine verringerte Querschnittsfläche des Führungsgehäuses, so daß das Operationsgebiet am Gehäuse vorbei für das Auge und zusätzlich für Instrumente zugänglich ist. Ferner bleibt der Führungsstift beim Verdrehen des Führungsgehäuses stehen, so daß die im Falle eines mitdrehenden Führungsstiftes auftretenden Nachteile vermieden werden. Daher ist eine genauere Zentrierung vorhanden und der Gewebekegel wird nicht auf Reibung beansprucht, so daß sich eine exakte Kegelgestalt ergibt.

Aus der DE-PS 29 49 278 ist zwar ein Konisator bekannt, der hinsichtlich der Beobachtbarkeit des Operationsgebietes ähnliche Geometrieverhältnisse aufweist. Es handelt sich hier jedoch nicht um einen Laser- sondern um einen Messerkonisator mit einem bandförmigen flexiblen Messer, das mit seinem in die Gebärmutterhalsöffnung einzuführenden Ende am Vorderende einer Stange und mit seinem entgegengesetzten Ende an einer radial von der Stange abstehenden Führung gehaltert ist. Somit liegt dieser Vorrichtung ein ganz anderes Prinzip zugrunde.

Die erfindungsgemäße Vorrichtung ist zwar in erster Linie zum kegelähnlichen Ausschneiden des der Scheide zugewandten Bereichs des Gebärmutterhalses gedacht. Prinzipiell kann sie jedoch auch an anderen Körperstellen und bei Tieren eingesetzt werden.

Zweckmäßige Ausgestaltungen der Erfindung sind in den Unteransprüchen angegeben.

Ausführungsbeispiele der Erfindung sowie die in den Unteransprüchen angegebenen zweckmäßigen Ausgestaltungen werden nun anhand der Zeichnung beschrieben. Es zeigen:

Figur 1 Eine erfindungsgemäße Vorrichtung in schematischer Darstellung bei ihrer Anwendung zum Ausschneiden. eines Gewebekegels aus dem der Scheide zugewandten Bereich des Gebärmutterhalses,

Fig. 2 die Vorrichtung nach Fig. 1 in Schrägansicht,

Fig. 3 die Vorrichtung gemäß den Fig. 1 und 2 im Querschnitt durch das Auslegerteil im das austrittsseitige Umlenkteil enthaltenden Bereich,

Fig. 4 den ausgeschnittenen Gewebekegel in Schrägansicht;

Fig. 5 eine weitere Ausführungsform der Erfindung in einer der Fig. 1 entsprechenden schematischen Darstellung und

Fig. 6 ein drittes Ausführungsbeispiel in einer der Fig. 2 entsprechenden Schrägansicht in Teildarstellung.

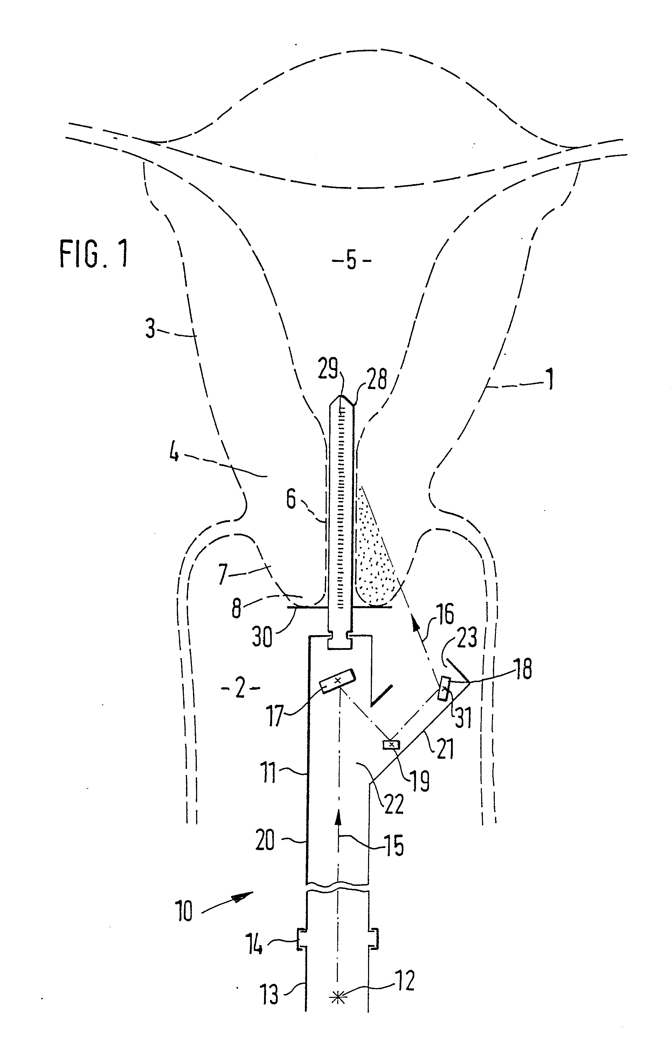

In Fig. 1 sind gestrichelt eine menschliche Gebärmutter 1 und daran anschließende Scheide 2 angedeutet. Die Gebärmutter 1 besteht aus dem Gebärmutterkörper 3 und dem der Scheide 2 zugewandten Gebärmutterhals 4. Der Gebärmutterhals 4 wird von einer einerseits in die Scheide 2 und andererseits in die Gebärmutterhöhle 5 mündenden Gebärmutteröffnung 6 durchdrungen. Der Gebärmutterhals 4 ragt einige Zentimeter in die Scheide 2 vor. Dieser unterste Abschnitt des Gebärmutterhalses 4 ist der Gebärmuttermund 7. Am Gebärmuttermund 7 befindet sich eine Übergangs- oder Transformationszone 8, an der die die Gebärmutterhöhle 5 auskleidende Gebärmutter schleimhaut in ein Deckgewebe übergeht, das den in die Scheide 2 ragenden Teil der Gebärmutter bedeckt. Diese Zone stellt einen Regenerationsbereich dar, in dem die Zelldifferenzierung stattfindet und von dem auch atypische Zellen ausgehen können, die nach einer gewissen Latenzzeit in bösartiges Wachstum übergehen können. Bei rechtzeitiger Diagnosestellung können diese präinvasiven Stadien des Gebärmutterhalskarzinoms durch Ausschneiden eines je nach Befund kleineren oder größeren Kegels entfernt werden, dessen Basisfläche der Scheide 2 und dessen Spitze der Gebärmutterhöhle 5 zugewandt ist.

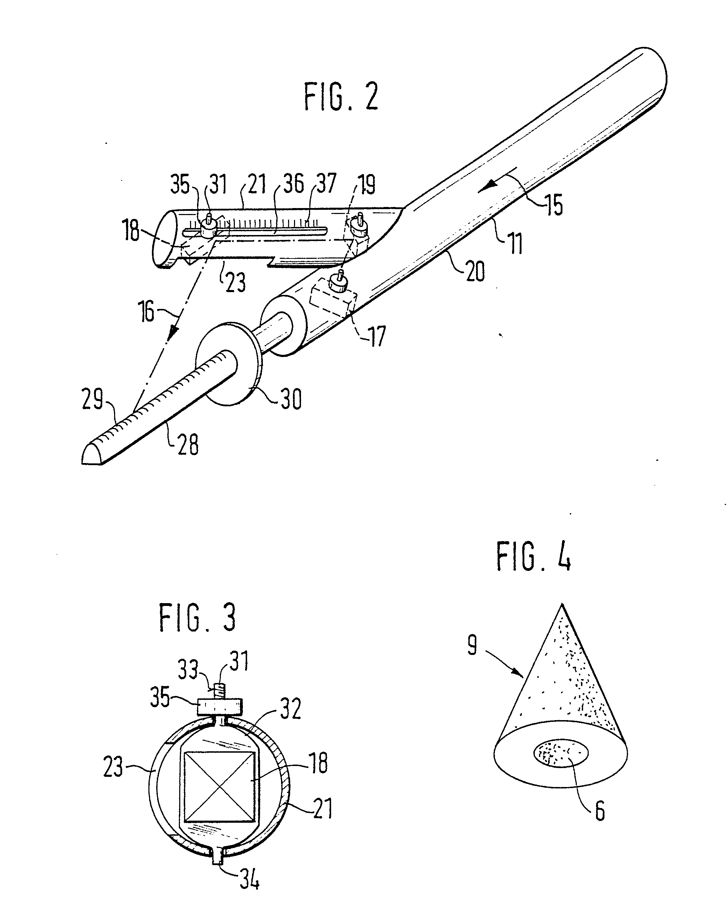

Zum Ausschneiden des in Fig. 4 gesondert dargestellten Gewebekegels 9 dient eine Vorrichtung 10. Diese weist ein Laserstrahl-Führungsgehäuse 11 auf, das mit einem eine Laser-Quelle 12 enthaltenden Laserstrahl-Erzeuger 13 verbunden ist. Dabei ist das Laserstrahl-Führungsgehäuse 11 verdrehbar mit der Laser-Quelle 12 bzw. dem Laserstrahl-Erzeuger 13 verbunden, wozu ein bei 14 schematisch angedeutetes Zwischenstück dient.

Der von der Laser-Quelle 12 kommende, strichpunktiert dargestellte Laserstrahl gelangt in Eintrittsrichtung 15 in das Führungsgehäuse 11 und verläßt dieses in Austrittsrichtung 16. Dabei verläuft die Austrittsrichtung 16 spitzwinkelig zur Eintrittsrichtung 15. Das Umlenken des Laserstrahls erfolgt mittels einer im Führungsgehäuse 11 angeordneten Umlenkeinrichtung, die ein den Laserstrahl aus der Eintrittsrichtung 15 weg lenkendes, eintrittsseitiges Umlenkteil 17 und ein den Laserstrahl in die Austrittsrichtung 16 lenkendes, austrittsseitiges Umlenkteil 18 enthält. Beim Ausführungsbeispiel nach den Fig. 1 und 2 befindet sich zwischen dem eintrittsseitigen Umlenkteil 17 und dem austrittsseitigen Umlenkteil 18 ein Zwischenumlenkteil 19, so daß der Laserstrahl vom eintrittsseitigen Umlenkteil 17 zum Zwischenumlenkteil 19 und von diesem anschließend zum austrittsseitigen Umlenkteil 18 gelenkt wird, so daß sich ein zickzackähnlicher Strahlengang ergibt.

In jedem Falle befindet sich das austrittsseitige Umlenkteil 18 mit Seitenabstand zum eintrittsseitigen Umlenkteil 17, wobei es in Eintrittsrichtung 15 des Laserstrahls gesehen je nach den örtlichen Gegebenheiten vor oder 11 hinter diesem angeordnet sein kann. Die Austrittsrichtung 16 verläuft so spitzwinkelig zur Eintrittsrichtung 15, daß der austretende Laserstrahl die über das eintrittsseitige Umlenkteil 17 hinaus verlängerte gedachte Linie schneidet.

Die Umlenkteile 17, 18, 19 der Umlenkeinrichtung werden in üblicher Weise insbesondere von entsprechend schräggestellten Spiegeln oder Prismen gebildet.

Das Führungsgehäuse 11 mit der Umlenkeinrichtung 17, 18, 19 ist um eine mit der Eintrittsrichtung 15 zusammenfallende und deshalb nicht besonders gekennzeichnete Drehachslinie drehbar. Dreht man das Führungsgehäuse 11 um diese Drehachslinie bzw. die Eintrittsrichtung 15, dreht sich die Umlenk einrichtung und somit der in Austrittsrichtung 16 austretende Laserstrahl mit. Bei dieser Drehbewegung überstreicht der entlang der Mantellinie eines Kegels verlaufende austretende Laserstrahl eine Kegelfläche, wobei die Drehachslinie die Kegelachse bildet und die Kegelbasisfläche der Umlenkeinrichtung zugewandt ist.

Bei der Anwendung der Vorrichtung 10 bringt man deren Führungsgehäuse 11 bis nahe vor den auszuschneidenden Gewebebereich, so daß der Laserstrahl frei auf die betreffende Körperstelle trifft. Beim Drehen des Führungsgehäuses 11 schneidet der Laserstrahl dann den Gewebekegel 9 aus. Beim aus Fig. 1 hervorgehenden Anwendungsbeispiel wird das Führungsgehäuse 11 koaxial zur Gebärmutterhalsöffnung 6 gedreht. Die halbe Längsschnittfläche des sich ergebenden Gewebekegels 9 ist in Fig. 1 gepunktet angedeutet.

Beim bevorzugten Ausführungsbeispiel weist das Führungsgehäuse 11 ein eintrittsseitiges Führungsrohr 20 und ein seitlich von diesem abstehendes, das austrittsseitige Umlenkteil 18 tragendes Auslegerteil 21 auf. Das Führungsrohr 20 ist einerseits, wie schon erwähnt, drehbar mit der Laser-Quelle 12 verbunden, so daß es und damit auch das Auslegerteil 21 bei der Operation relativ zur feststehenden Laser-Quelle verdreht werden kann. Im Bereich des entgegengesetzten Endes des Führungsrohrs 20, in dem der eintretende Laserstrahl verläuft, ist das eintrittsseitige Umlenkteil 17 angeordnet. Damit der vom eintrittsseitigen Umlenkteil 17 abgelenkte Laserstrahl aus dem Führungsrohr 20 austreten kann, weist dieses ein entsprechend angeordnetes Seitenfenster 22 auf. Beim Ausführungsbeispiel nach den Fig. 1 und 2 wird der Laserstrahl vom eintrittsseitigen Umlenkteil 17 ein Stück weit zurückgelenkt, und zwar zum Zwischenumlenkteil 19, das sich im Bereich des Übergangs vom Führungsrohr 20 zum Auslegerteil 21 befindet. Anschließend durchläuft der Laserstrahl das Auslegerteil 21 in schräg nach vorne außen gerichteter Richtung bis zum austrittsseitigen Umlenkteil 18. Auf Grund dieser Strahlengeometrie steht das Auslegerteil 21 spitzwinkelig vom Führungsrohr 20 ab. Auch das Auslegerteil 21 kann von einem Rohrstück gebildet werden, das eine Austrittsöffnung 23 für den Laserstrahl besitzt.

Das Führungsgehäuse 11 besteht aus Metall, wobei das Auslegerteil 21 seitlich an das Führungsrohr 20 angeschweißt sein kann.

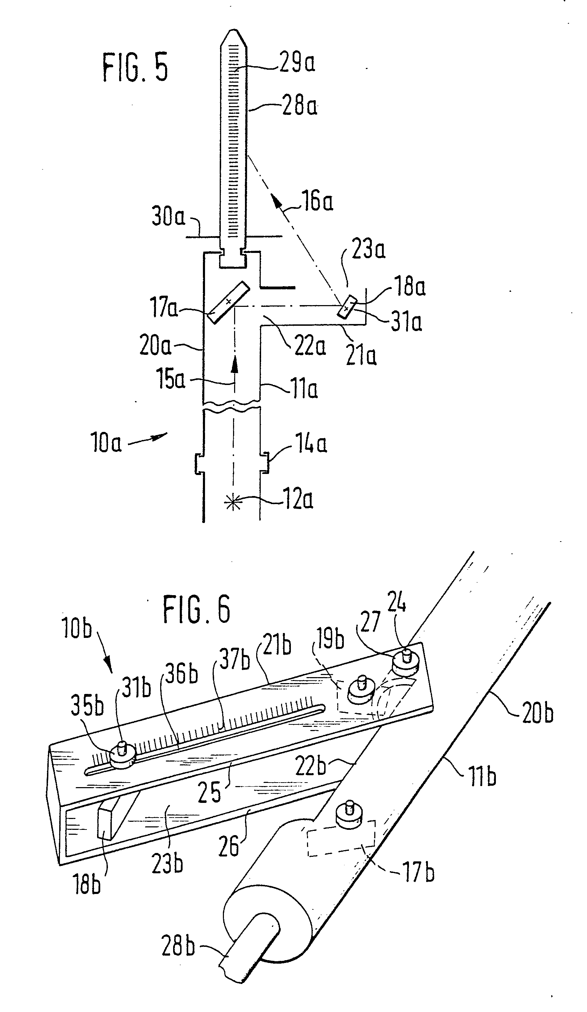

Bei dem ansonsten übereinstimmenden Ausführungsbeispiel nach Fig. 5 ist das Zwischenumlenkteil 19 weggelassen. In diesem Falle lenkt das eintrittsseitige Umlenkteil 17a den Laserstrahl unmittelbar zum austrittsseitigen Umlenkteil 18a. Dabei ist der Laserstrahl zwischen den beiden Umlenkteilen 17a und 18a etwa rechtwinkelig zur Eintrittsrichtung 15a gerichtet. Dementsprechend steht das Auslegerteil 21a etwa rechtwinkelig vom Führungsrohr 20a ab. Wegen der ansonsten vorhande-nen Übereinstimmung wird das Ausführungsbei-spiel nach Fig. 5 nicht weiter beschrieben. Die entsprechenden Teile sind mit den gleichen Bezugsziffern unter Hinzufügen eines "a" bezeichnet.

Während bei den Ausführungsbeispielen nach den Fig. 1 bis 3 und 5 das Auslegerteil 21 bzw. 21a starr vom Führungsrohr 20 absteht, ist es gemäß dem in Fig. 6 dalgestellten Ausführungsbeispiel auch möglich, das das austrittsseitige Umlenkteil 18b tragende Auslegerteil 21b schwenkbar mit dem Führungsrohr 20b zu verbinden, so daß der Winkel zwischen dem Führungsrohr und dem Auslegerteil veränderbar ist. Hierzu ist das beispielsweise von einem austrittsseitig offenen U-Profilstück gebildete Auslegerteil 21b über eine Schwenkachse 24 am Führungsrohr 20b gelagert. Es versteht sich, daß das Auslegerteil 21b so bemaßt ist, daß das Verschwenken nicht behindert wird. Daher ist beim dargestellten Ausführungsbeispiel der Abstand zwischen den beiden Schenkeln 25, 26 des Auslegerteils 21b größer als der Durchmesser des Führungsrohrs 20b, so daß das Auslegerteil am angelenkten Ende das Führungsrohr übergreifen kann. Zum Feststellen des Auslegerteils 21b in der jeweiligen Schwenklage kann die Schwenkachse 24 beispielsweise von einem Gewindestift gebildet werden, der das Auslegerteil 21b und das Führungsrohr 20b durchdringt und einerseits ein fest mit ihm verbundenes Klemmstück und andererseits eine Gewindemutter 27 trägt, bei deren Festziehen ein Verklemmen stattfindet. Mit Ausnahme der schwenkbaren Anordnung des Auslegerteils 21b entspricht das Ausführungsbeispiel nach Fig. 6 dem Ausführungsbeispiel nach den Fig. 1 bis 3, so daß im übrigen auf seine Beschreibung verzichtet wird.

Ob man eine Vorrichtung mit oder ohne Zwischenumlenkteil 19 verwendet, hängt von den örtlichen Gegebenheiten an der Operationsstelle ab. Handelt es sich hier um den Gebärmuttermund, liegen beengte Platzverhältnisse vor, so daß die weniger seitlich ausladende Anordnung nach den Fig. 1 und 2 günstiger sein kann.

Bei dem Ausführungsbeispiel nach Fig. 6 ist zwar eine bestmögliche Anpassung an die jeweiligen Platzverhältnisse möglich. In jeder Schwenklage des Auslegerteils 21b(müssen) kann es jedoch erforderlich sein, alle Umlenkteile 17b, 18b und 19b neu einzujustieren. Demgegenüber kann bei starr angeordnetem Auslegerteil das eintrittsseitige Umlenkteil 17 bzw. 17a und das gegebenenfalls vorhandene Zwischenumlenkteil 19 seine Lage stets unverändert beibehalten, und zwar unabhängig von den Maßen des auszuschneidenden Gewebekegels 9, dessen Abmessungen sich durch Verstellen des austrittsseitigen Umlenkteils 18 bzw. 18a allein variieren lassen, wie noch beschrieben werden wird.

Bei jedem Ausführungsbeispiel weist die Vorrichtung 10; 10a; 10b am in Gebrauchslage der Operationsstelle zugewandten Ende einen zur mit der Eintrittsrichtung 15; 15a zusammenfallenden Drehachslinie koaxialen Führungsstift 28; 28a; 28b od.dgl. auf, der in axialer Richtung vorsteht und in eine im zu entfernenden Gewebeteil enthaltene Körperöffnung, insbesondere in die Gebärmutterhalsöffnung 6, steckbar ist, so daß der Führungsstift 28; 28a; 28b od.dgl. das Führungsgehäuse 11; 11a; 11b bei seinem Verdrehen zentriert. Der Führungsstift 28; 28a; 28b steht also in axialer Richtung vor das das eintrittsseitige Umlenkteil 17; 17a; 17b ent haltende Ende des Führungsrohrs 20; 20a; 20b vor und kann bei der Operation in die betreffende Körperöffnung 6 gesteckt werden. Die Körperöffnung 6 bildet somit eine Lageröffnung für den Führungsstift 28; 28a; 28b und somit für das ganze Führungsgehäuse 11; 11a; 11b, so daß dieses nicht anderweitig zentriert und nur gedreht werden muß.

Der Führungsstift 28; 28a; 28b od.dgl. steht zweckmäßigerweise bis über die maximal einstellbare Kegelhöhe (wird noch beschrieben) axial vor. Dies bringt nicht nur eine entsprechend gute Zentrierung in der Körperöffnung 6, sondern noch weitere Vorteile mit sich. Besteht nämlich der Führungsstift 28; 28a; 28b od.dgl. aus den Laserstrahl nicht durchlassendem und nicht reflektierendem Material, endet der Laserstrahl unabhängig von den Abmessungen des Gewebekegels 9 stets am Führungsstift 28; 28a; 28b od.dgl., so daß der Laserstrahl nicht weiter nach innen in das Gewebe ein-dringen und unerwünschte Verletzungen hervorru-fen kann Ferner kann der Führungsstift zum leichteren präoperativen Einstellen der Richtung 16; 16a des austretenden Laserstrahls dienen. Vor dem Eingriff werden nämlich auf Grund einer mikroskopischen Betrachtung des äußeren Teils des Gebär-muttermundes die Abmessungen des auszuschnei-denden Kegels bestimmt. Sodann wird ebenfalls vor der Operation und außerhalb der Patientin die Umlenkeinrichtung so justiert, daß die Lage und Richtung des austretenden Laserstrahls den gewünschten Kegel ergeben. Hierzu kann mit einem Pilot-Laser (beispielsweise ein Helium-Neon-Laser) gearbeitet werden. Dieser Pilot-Laserstrahl trifft auf den Führungsstift 28; 28a; 28b auf, wobei die Auftreffstelle sichtbar ist. Die Axiallage der Auftreffstelle am Führungsstift entspricht der Kegelhöhe, und die Verbindungslinie zwischen der Auftreffstelle und dem austrittsseitigen Umlenkteil 18 entspricht dem Konuswinkel, so daß auch die Größe der Kegelbasisfläche erkannt werden kann.

Das präoperative Justieren der Umlenkeinrichtung auf die gewünschten Kegelabmessungen läßt sich dadurch erleichtern, daß man am Führungsstift 28; 28a; 28b od.dgl. eine in axialer Richtung verlaufende Maßskala 29; 29a anbringt. Man kann dann die im voraus berechnete Kegelhöhe ablesen.

Bei Durchführung des Eingriffs (der Pilot-Laser ist jetzt abgeschaltet) wird der Führungsstift 28; 28a; 28b in die Gebärmutter eingeführt, und zwar so weit, daß der Nullpunkt der Maßskala 29 in gleicher Höhe wie die Basis des auszuschneidenden Konus, also wie die Oberfläche des Gebärmuttermundes steht. Hierbei kann die Handhabung weiter dadurch vereinfacht werden, daß ein die Einstecktiefe des Führungsstiftes 28; 28a; 28b od.dgl. in die Körperöffnung 6 begrenzendes Anschlagteil 30; 30a, zweckmäßigerweise ein am Führungsstift od.dgl. angeordneter Anschlagring, vorhanden ist, an dem der Nullpunkt der Maßskala 29; 29a liegt. Das Anschlagteil sollte wie der Führungsstift od.dgl. und das Führungsgehäuse aus einem geeigneten Metall bestehen.

Anschließend wird dann die Arbeitslaser-Quelle 12 eingeschaltet und das Führungsgehäuse 11; 11a; 11b einmal oder mehrmals um 360° gedreht. Dabei ist vorgesehen, daß sich nur das Führungsgehäuse mit der Umlenkeinrichtung dreht, während der Führungsstift od.dgl. stehenbleibt. Hierzu ist das Führungsgehäuse verdrehbar mit dem Führungsstift od.dgl. verbunden. Die Drehbewegung muß mit einer solchen Geschwindigkeit durchgeführt werden, daß der Laserstrahl bis zum Führungsstift 28; 28a; 28b od.dgl. vordringen kann. Gegebenenfalls ist die Drehung zu wiederholen, bis der Kegel vom Gebärmuttermundrest abgelöst werden kann. Persistierende kleinere Blutungen am Stumpf des Gebärmuttermundes können gelasert oder koaguliert werden.

Zum Einstellen der gewünschten Lage und Orientierung des austretenden Laserstrahls ist folgendes vorgesehen:

Zur Veränderung des Kegelwinkels ist das austrittsseitige Umlenkteil 18; 18a; 18b schwenkbar und in der jeweiligen Schwenkstellung feststellbar angeordnet. Die betreffende Schwenkachse 31; 31a; 31b steht senkrecht zur Ebene des Strahlengangs und kann beispielsweise dadurch gebildet werden, daß das austrittsseitige Umlenkteil bzw. ein dieses haltender Rahmen 32 od.dgl. zwei am Führungsgehäuse bzw. an dem Auslegerteil 21; 21a; 21b gelagerte Lagerzapfen 33, 34 (siehe Fig. 3, wobei dies auch für die anderen Ausführungsbeispiele gilt) aufweist. Einer der Lagerzapfen, beispielsweise der Lagerzapfen 33, trägt ein Außengewinde, so daß auf den aus dem Führungsgehäuse vorstehenden Bereich des Lagerzapfens 33 eine Feststellmutter 35; 35b aufgeschraubt werden kann. Löst man die Feststellmutter, kann das betreffende Umlenkteil von außen her beispielsweise durch Ergreifen des Lagerzapfens 33 verschwenkt werden. Zieht man die Feststellmutter 35 dann fest, wird das Umlenkteil 18; 18a; 18b bzw. dessen Rahmen 32 od.dgl. von innen her gegen die Wandung des Führungsgehäuses gezogen, bis eine feste Verspannung vorliegt.

Der Radius der Kegelbasisfläche läßt sich dagegen dadurch verstellen, daß das austrittsseitige Umlenkteil 18; 18a; 18b mit veränderbarem und feststellbarem Abstand zur mit der Eintrittsrichtung 15; 15a des Laserstrahls zusammenfallenden Drehachslinie am Führungsgehäuse angeordnet ist. Dabei erfolgt diese Längsverstellung des austrittsseitigen Umlenkteils genauer gesagt in der Richtung des Laserstrahls, in der dieser auf das austrittsseitige Umlenkteilzuläuft. Bei den Ausführungsbeispielen nach den Fig. 1 bis 3 und 6 erfolgt die Verstellung also längs der Verbindungslinie zwischen dem austrittsseitigen Umlenkteil 18; 18b und dem Zwischenumlenkteil 19; 19b, während bei dem Ausführungsbeispiel nach Fig. 5 das Verstellen längs der Verbindungslinie zwischen dem eintrittsseitigen Umlenkteil 17a und dem austrittsseitigen Umlenkteil 18a durchgeführt wird. Unabhängig von der Position des austrittsseitigen Umlenkteils können die anderen Umlenkteile dann ihre Position und Drehlage unverändert beibehalten.

Somit kann man allein durch Verdrehen und eine Längsver schiebung des austrittsseitigen Umlenkteils die genau gewünschte, frei wählbare Kegelform erhalten.

Zum Verschieben des austrittsseitigen Umlenkteils am Führungsgehäuse bzw. an dessen Auslegerteil ist an diesem eine geeignete Führungseinrichtung vorhanden, beispielsweise indem die Lagerachse 31; 31a; 31b bzw. die diese bildenden Lagerzapfen 33, 34 in einem in Verschieberichtung verlaufenden Schlitz 36; 36b geführt sind. Zum Verschieben des austrittsseitigen Umlenkteils löst man wieder die Feststellmutter 35; 35b, die dann bei Erreichen der gewünschten Schlitzposition wieder festgezogen wird.

Wie aus der Zeichnung in diesem Zusammenhang noch hervorgeht, kann das Führungsgehäuse im Bereich des austrittsseitigen Umlenkteils eine von außen her ablesbare Maßskala 37; 37b aufweisen, an der sich der Abstand zur Drehachslinie bzw. zum im Strahlengang davor angeordneten Umlenkteil ablesen läßt.

Das eintrittsseitige Umlenkteil 17; 17a; 17b und das gegebenenfalls vorhandene Zwischenumlenkteil 19; 19b sind ebenfalls um eine rechtwinkelig zur Ebene des Strahlengangs stehende Achse verdrehbar und feststellbar. Dies kann in gleicher Weise wie oben für das austrittsseitige Umlenkteil beschrieben erfolgen. Dabei muß die Drehlage dieser Umlenkteile jedoch nur einmal einjustiert werden und kann dann unverändert bleiben, so daß dieser Justiervorgang fabrikseitig erfolgen kann.

Nachzutragen ist noch, daß die Vorrichtung dem Anwendungsgebiet entsprechend möglichst klein bauen sollte. Das im wesentlichen das Führungsgehäuse bildende Führungsrohr 20; 20a; 20b kann deshalb einen sehr kleinen Durchmesser von etwa 0,5 - 1 cm aufweisen.

Bei einer nicht dargestellten Variante läßt sich die Länge des Führungsrohres verändern, indem man das Führungsrohr teleskopartig aus ineinandergesteckten Rohrstücken zusammensetzt.

Weitere Möglichkeiten sind folgende:

Die der Verschiebung des austrittsseitigen Umlenkteils zugeordnete Maßskala 37; 37b könnte durch eine elektronische Anzeige ersetzt werden, die von einem Rechner gesteuert wird. Dieser Rechner könnte unter Berücksichtigung der Drehlage, d. h. des Einstellwinkels des austrittsseitigen Umlenkteils, den Durchmesser der Kegelbasisfläche und die Kegelhöhe in bezug auf die am Führungsstift 28; 28a; 28b angebrachte Nullmarkierung berechnen.

Ferner könnte in den Führungsstift od.dgl. eine Meßeinheit eingebaut werden, die das Auftreffen des Laserstrahls auf den Führungsstift anzeigt und dem Operateur einen Hinweis auf die erforderliche Schnelligkeit der Drehbewegung gibt. Dazuhin könnte man die Drehbewegung des Gerätes nicht von Hand ausführen, sondern durch einen Motor antreiben, der in Abhängigkeit einer solchen Meßeinheit am Führungsstift arbeitet.

**Ansprüche**

1. Vorrichtung zum operativen Entfernen eines kegelähnliche Gestalt aufweisenden und eine Öffnung enthaltenden Gewebeteils aus einem menschlichen oder tierischen Körper durch Ausschneiden mittels eines Laserstrahls,

- mit einem Laserstrahl-Führungsgehäuse, das ein Rohr, durch das der eintretende Laserstrahl verläuft, sowie ein den Laserstrahl aus seiner Eintrittsrichtung weglenkendes eintrittsseitiges Umlenkteil und ein mit Seitenabstand zu diesem angeordnetes, den Laserstrahl in seine Austrittsrichtung lenkendes austrittsseitiges Umlenkteil aufweist, so daß beim Verdrehen des Führungsgehäuses um die Eintrittsrichtung der austretende Laserstrahl eine Kegelmantelfläche überstreicht - und mit einem in die Öffnung steckbaren und das Führungsgehäuse beim Verdrehen zentrierenden Führungsstift,

**dadurch gekennzeichnet,**

- daß das Führungsgehäuse (11; 11a; 11b;) ein seitlich vom Rohr (20; 20a; 20b) abstehendes Auslegerteil (21; 21a; 21b) aufweist, an dem das austrittsseitige Umlenkteil (18; 18a; 18b) angeordnet ist und

- daß der Führungsstift (28; 28a; 28b) und das Führungsgehäuse (11; 11a; 11b) gegeneinander verdrehbar sind.

2. Vorrichtung nach Anspruch 1, dadurch gekennzeichnet, daß der Führungsstift (28; 28a; 28b) aus den Laserstrahl nicht reflektierendem Material besteht.

3. Vorrichtung nach Anspruch 1 oder 2, dadurch gekennzeichnet, daß der Führungsstift (28; 28a; 28b) eine in axialer Richtung verlaufende Maßskala (29, 29a) trägt.

4. Vorrichtung nach einem der Ansprüche 1 bis 3, dadurch gekennzeichnet, daß am Führungsstift (28; 28a; 28b) ein seine Einstecktiefe in die Öffnung begrenzendes Anschlagteil (30; 30a) angeordnet ist.

5. Vorrichtung nach einem der Ansprüche 1 bis 4, dadurch gekennzeichnet, daß das austrittsseitige Umlenkteil (18; 18a; 18b) zum Verändern des Kegelwinkels schwenkbar und in der jeweiligen Schwenkstellung feststellbar und, oder zum Verändern des Radius der Kegelbasisfläche mit veränderbarem und feststellbarem Abstand zur mit der Eintrittsrichtung zusammenfallenden Drehachslinie (15; 15a; 15b) am Führungsgehäuse (11; 11a; 11b) angeordnet ist.

6. Vorrichtung nach Anspruch 5, dadurch gekennzeichnet, daß das Führungsgehäuse (11; 11a; 11b) im Bereich des austrittsseitigen Umlenkteils (18; 18a; 18b) eine von außen her ablesbare Maßskala (37; 37b) zum Ablesen des Abstandes zur Drehachslinie aufweist.

7. Vorrichtung nach einem der Ansprüche 1 bis 6, dadurch gekennzeichnet, daß zwischen dem eintrittsseitigen Umlenkteil (17, 17b) und dem austrittsseitigen Umlenkteil (18; 18a) ein Zwischenumlenkteil (19; 19a) angeordnet ist, so daß sich ein zick-zack-ähnlicher Strahlengang ergibt.

8. Vorrichtung nach einem der Ansprüche 1 bis 7, dadurch gekennzeichnet, daß das Auslegerteil (21) spitzwinkelig vom Rohr (20) absteht.

9. Vorrichtung nach einem der Ansprüche 1 bis 8, dadurch gekennzeichnet, daß das Auslegerteil (21b) schwenkbar mit dem Rohr (20b) verbunden ist.

10. Vorrichtung nach einem der Ansprüche 1 bis 9, dadurch gekennzeichnet, daß das Rohr in der Länge teleskopartig verstellbar ist.

FIG. 1

# FIG. 2

# FIG. 3

# FIG. 4

FIG. 5

FIG. 6